# EUROPEAN PATENT SPECIFICATION

(11) **EP 4 129 284 B1**
(45) Date of publication and mention of the grant of the patent: **18.12.2024**
(21) Application number: 21189800.2
(22) Date of filing: 05.08.2021
(51) Int. Cl.: A61K 31/4178, A61P 31/10, A01N 43/653, A01P 3/00

(54) **SERTACONAZOL FOR PROVIDING A SPORISTATIC AND/OR SPORICIDAL EFFECT**
SERTACONAZOL ZUR BEREITSTELLUNG VON SPORISTATISCHER UND/ODER SPORIZIDER WIRKUNG
SERTACONAZOLE POUR FOURNIR UN EFFET SPORISTATIQUE ET/OU SPORICIDE

(43) Date of publication of application: 08.02.2023
(73) Proprietor: Dr. Pfleger Arzneimittel GmbH, 96052 Bamberg (DE)
(72) Inventor: Hipler, Uta-Christina, 96052 Bamberg (DE); Wiegand, Cornelia, 96052 Bamberg (DE); Schwantes, Ulrich, 96052 Bamberg (DE); Neumeister, Claudia, 96052 Bamberg (DE); Götz, Marcus Rudolf, 96052 Bamberg (DE)
(74) Representative: Eisenführ Speiser

(56) References cited:
- US-A- 5 135 943
- US-A1- 2007 092 547
- US-A1- 2008 220 103
- LAURENT ALEXIS ET AL: "Production of Trichophyton rubrum microspores in large quantities and its application to evaluate amorolfine/azole compound interactions in vitro", MYCOSES, vol. 60, no. 9, 8 May 2017 (2017-05-08), GB, pages 581 - 586, XP055880545, ISSN: 0933-7407, Retrieved from the Internet <URL:https://api.wiley.com/onlinelibrary/tdm/v1/articles/10.1111%2Fmyc.12632> [retrieved on 20220118], DOI: 10.1111/myc.12632
- CARRILLO-MUÑOZ ALFONSO J. ET AL: "Sertaconazole Nitrate Shows Fungicidal and Fungistatic Activities against Trichophyton rubrum, Trichophyton mentagrophytes, and Epidermophyton floccosum, Causative Agents of Tinea Pedis", ANTIMICROBIAL AGENTS AND CHEMOTHERAPY, vol. 55, no. 9, 11 July 2011 (2011-07-11), US, pages 4420 - 4421, XP055880649, ISSN: 0066-4804, DOI: 10.1128/AAC.00219-11

## Description

The present invention relates to sertaconazol for use in the prevention and/or treatment of a fungal infection, wherein the prevention and/or treatment comprises or is providing a sporistatic and/or sporicidal effect against the spores of the fungus causing the fungal infection.

The present invention also relates to a non-therapeutic use of sertaconazol, or a salt thereof, for providing a sporistatic and/or sporicidal effect against the spores of a fungus.

Nail disorders as well as skin disorders, particularly fungal nail or skin disorders, are a physical as well as a mental stress for humans and usually strongly reduce the quality of life of the patients. Often these fungal disorders, i.e. fungal infections, appear in parallel.

Fungal infections can affect the upper layers as well as the deeper layers of the skin. Often, infections of yeasts or mold may also occur, in case the immune system is weakened, e.g. as an opportunistic infection. Likewise, onychomycosis can be caused by several fungi such as dermatophytes, yeasts or mold.

The total number of fungal infections is continuously increasing, whereas an alarming shift from anthropophilic dermatophytes such as *Trichophyton rubrum* or *Trichophyton interdigitale* to an increasing prevalence of zoophilic dermatophytes such as *Trichophyton benhamiae* or *Trichophyton mentagrophytes* can be observed.

Furthermore, several resistant strains have formed in recent times, thus providing further challenges in efficiently treating fungal infections.

Moreover, another difficulty in efficiently treating fungal infections is that fungi may produce spores, which are considered as inactive entities, which have reduced their metabolism to a minimum. In some cases such spores can survive long times without water and under rough conditions such as heat, cold, radiation, chemical agents or extreme pH values. Usually, spores have a thickened cell wall, rendering them even further resistant to therapeutic interventions.

When reactivating, the cell wall of the spores becomes more permeable to various substances and water. Thus, the spores get access to energy resources such as glucose and further nutrients as well as water. With increasing energy resources and nutrients, the spores produce new cell components and increase their volume by growth and water absorption (outgrowth).

In many cases, an active fungal infection may be treated with the common substances. However, the spores are typically not affected by such treatment and remain in the infected tissue. After short time, these spores become active and again infect the tissue. Thus, frequently, a reoccurring fungal infection is observed.

US2007/092547 and US2008/220103 disclose antifungal mixtures which have sporistatic or sporicidal effects.

Consequently, a strong need of novel antimycotic substances exists, which can be used against the variety of fungi and particularly their spores.

Such antimycotic, sporistatic and/or sporicidal substances need to be characterized with regard to their activity. Furthermore, to be applied in vivo, such substances need to be particularly effective on human (or animal) tissue cells and simultaneously must not provide any or any substantial harm to these cells or to the entire organism, when applied systemically.

A few substances have already been identified and used. However, as all of such substances provide side effects in a certain concentration or after a certain duration of treatment, there is a persistent need to identify antimycotic, sporistatic and/or sporicidal substances with a higher efficacy, such that they can be applied in lower doses, which reduces the risk of side effects.

The primary object of the present invention was thus to provide a novel treatment, which efficiently targets these spores and - at the same time - does not (substantially) provide harm for the cells or organism to be treated.

The primary object of the present invention is solved by sertaconazol, or a salt thereof, preferably sertaconazol nitrate, for use in the prevention and/or treatment of a fungal infection, wherein the prevention and/or treatment comprises or is providing a sporistatic and/or sporicidal effect against the spores of the fungus causing the fungal infection.

The term "sertaconazol" as used herein is meant to be understood such that it encompasses the substance sertaconazol itself, as well as any possible salts thereof, such as preferably sertaconazol nitrate.

The term "wherein the prevention and/or treatment comprises or is providing a sporistatic and/or sporicidal effect" as used herein is meant to be understood such that providing the sporistatic and/or sporicidal effect is a necessary part of the prevention and/or treatment itself. Therefore, the prevention and/or treatment is fully or partly directed to evoking such an effect. Mere suitability of the antimycotic agent for providing such an effect or a coincidental provision of the effect in a treatment exclusively directed to another purpose is thus not sufficient to fall under said term.

As described herein, many therapies do not provide an effect on the spores of the fungus causing the fungal infection and thus, after a short time, the infection typically reoccurs. Consequently, patients, which have recently undergone a treatment of a fungal infection, are an example for patients in the risk of suffering from an infection. Thus, the term "prevention" as used herein comprises the prevention of a reoccurrence of a fungal infection, i.e. a new outburst of the infection after a previous fungal infection has been treated.

Furthermore, a fungal infection may be caused by exposure to spores of the fungus, which then reactivate and provide the growth and reproduction of the fungus. Consequently, patients with (assumed) exposure to spores of a fungus are an example for patients in the risk of suffering from an infection. Therefore, the term "prevention" as used herein comprises the prevention of an infection by (assumed) exposure to spores of a fungus.

As described herein, the prevention and/or treatment comprises or is providing a sporistatic and/or sporicidal effect. In this way, the prevention and/or treatment causes a long-lasting effect, as it is (at least partly) directed to the spores of the fungus causing the fungal infection. Thus, preferably, the prevention and/or treatment is directed to a long-term effect, i.e. a long-term prevention and/or a long-term treatment.

Furthermore, the group of patients exposed to prevention and/or treatment preferably comprises patients with an active fungal infection and patients, which are in the risk of suffering from an infection (either a novel infection, e.g. caused by exposure to spores, or a reoccurring infection). Particularly preferably, the group of patients comprises or consists of patients, which are in the risk of suffering from an infection (either a novel infection, e.g. caused by exposure to spores, or a reoccurring infection).

It was surprisingly found that the antimycotic drug sertaconazol, which was currently only known for a fungistatic and fungicidal effect, can also provide a sporistatic and/or sporicidal effect against the spores of fungi. This was particularly surprising, as (as described above) different requirements are raised for an active substance depending on whether fungal cells with an active metabolism or spores with a thickened cell wall, a reduced or inactive metabolism and further resistance to rough conditions are to be targeted. Consequently, the ability of a substance to cause a fungistatic and/or fungicidal effect does not allow the conclusion that the same substance does also cause a sporistatic and/or sporicidal effect. This is further underlined by the high number of re-occurring fungal infections, in which a substance with known fungistatic and/or fungicidal effect is used, however, the spores easily survive the respective treatment.

As shown in Example 2, sertaconazol is able to reduce and to inhibit the outgrowth of fungal spores and simultaneously does not harm the cells it is applied to or influence the cell viability. Furthermore, sertaconazol does not induce the release of pro-inflammatory markers such as interleukins IL-1α, IL-6 or IL-8.

Typically, the terms "prevention" and "treatment" as used herein do not necessarily require a total prevention of a fungal infection or a complete recovery. Instead, these terms preferably also include any partial prevention or treatment as long as a beneficial effect is provided or the extent of the (present or future) infection is reduced.

It is preferred in the treatment or prevention according to the invention that the fungus causing the fungal infection belongs to the group consisting of the genera *Trichophyton, Epidermophyton, Microascus* or *Candida.*

Furthermore, it is preferred in the treatment or prevention according to the invention that the fungus causing the fungal infection belongs to the group consisting of *Trichophyton rubrum, Trichophyton soudanense, Trichophyton interdigitale, Trichophyton mentagrophytes, Epidermophyton floccosum, Scopulariopsis brevicaulis, Candida albicans* and *Candida parapsilosis,*
particularly preferably from the group consisting of *Trichophyton mentagrophytes, Scopulariopsis brevicaulis, Candida albicans* and *Candida parapsilosis.*

It was surprisingly found that sertaconazol is particularly effective against these fungi. Further surprisingly, the effect of sertaconazol is superior to the effect of common other antimycotic substances for which a sporistatic and/or sporicidal effect was already known (see Examples 1 and 2).

Preferably, the prevention and/or treatment according to the invention comprises applying a composition comprising sertaconazol, or a salt thereof, preferably sertaconazol nitrate, in a concentration of at least 0.0001 wt.-%, preferably at least 0.001 wt.-%, particularly preferably at least 0.01 wt.-%, based on the total weight of the composition.

Preferably, the prevention and/or treatment according to the invention comprises applying a composition comprising sertaconazol, or a salt thereof, preferably sertaconazol nitrate, in a concentration of at least 0.1 wt.-%, preferably 0.5 wt.-%, particularly preferably 1 wt.-%, further preferably 1.25 wt.-%, even further preferably 1.5 wt.-% based on the total weight of the composition.

It was surprisingly found that sertaconazol is effective already in such low amounts. These amounts are rather low, compared to common other antimycotic substances for which a sporistatic and/or sporicidal effect was already known (see Examples 1 and 2). Thus, when used in such low amounts, the risk of side effects of the treatment is particularly reduced. Typically, pharmaceutical compositions to be used in a prevention or treatment according to the invention can, for example, be present in the form of solutions, suspensions, emulsions, tinctures, gels, creams, ointments, plasters comprising a pharmaceutical composition according to the invention, sprays, nail polishes and lacquers.

The pharmaceutical composition to be used in a prevention or treatment according to the invention can comprise additional components. These components are usually such that are typical for pharmaceutical compositions. Such components may comprise or consist of one or more ingredients of the following groups: fillers (e.g. cellulose, calcium carbonate), plasticizers and trickling substances (e.g. talc, magnesium stearate), coatings (e.g. polyvinylacetatphtalate, hydroxypropylmethylcellulosephtalate), softeners (e.g. triethylcitrate, dibutylphtalate), substances for granulation (lactose, gelatine), retardation (e.g. poly(meth)acrylicacidmethyl/ethyl/2-trimethyl-aminoethyl ester co-polymerisates in dispersion, vinylacetate/crotonic acid co-polymerisates), compaction (e.g. micro crystalline cellulose, lactose), solvents, suspension or dispersing agents (e.g. water, ethanol), emulsifiers (e.g. cetyl alcohol, lecithin), substances for changing rheological properties (silicondioxide, sodium alginate), substances for microbial stabilisation (e.g. benzalkonium chloride, potassium sorbate), preservatives and antioxidants (e.g. DL-alphatocopherol, ascorbic acid), substances for changing the pH value (lactic acid, citric acid), propellants and inert gases (e.g. fluorinated chlorinated hydrocarbons, carbon dioxide) dyes (iron oxides, titanium dioxide), ointment raw materials (e.g. paraffins, beeswax), and others as they appear in technical literature (e.g. Schmidt, P. C. et al., Wirk- und und Hilfsstoffe für Rezeptur, Defektur und Großherstellung 1999, or Bauer, K. H. et al., Lehrbuch der Pharmazeutischen Technologie 2006, 8. Auflage).

In the prevention and/or treatment according to the invention, sertaconazol or a salt thereof, may be applied in any way. Thus, the application may be systemic and/or local. Preferably, the prevention and/or treatment according to the invention comprises a local application of sertaconazol, or a salt thereof, preferably sertaconazol nitrate, wherein preferably the site of infection is targeted. Preferably, the local application is a dermal application or an application on the nail of a subject.

Further, the present invention relates to the non-therapeutic use of sertaconazol, or a salt thereof, preferably sertaconazol nitrate, for providing a sporistatic and/or sporicidal effect against the spores of a fungus.

Such a non-therapeutic use may be any use in which fungi or, respectively, their spores are to be removed or reduced. Such a use may be any use as disinfectant, e.g. for disinfecting tools or machines in the medical field or for cleaning rooms in or surfaces on which fungal presence shall be efficiently reduced. Furthermore, such a use can also be related to (industrial) plant growth.

What was said above with regard to sertaconazol or its use in the prevention and/or treatment according to the invention, applies to the non-therapeutic use accordingly, as far as applicable.

Thus, it is preferred that in the non-therapeutic use, the fungus is selected from the group consisting of fungi.

Particularly preferably, the fungus is selected from the group consisting of the genera *Trichophyton, Epidermophyton, Microascus* or *Candida.*

Furthermore, it is preferred that the fungus is selected from the group consisting of *Trichophyton rubrum, Trichophyton soudanense, Trichophyton interdigitale, Trichophyton mentagrophytes, Epidermophyton floccosum, Scopulariopsis brevicaulis, Candida albicans* and *Candida parapsilosis,*
particularly preferably from the group consisting of *Trichophyton mentagrophytes, Scopulariopsis brevicaulis, Candida albicans* and *Candida parapsilosis.*

It is further preferred that in the use according to the invention, sertaconazol or a salt thereof, preferably sertaconazol nitrate, is present in a composition in a concentration of at least 0.0001 wt.-%, preferably at least 0.001 wt.-%, particularly preferably at least 0.01 wt.-%, based on the total weight of the composition.

It is further preferred that in the use according to the invention, sertaconazol or a salt thereof, preferably sertaconazol nitrate, is present in a composition in a concentration of at least 0.1 wt.-%, preferably 0.5 wt.-%, particularly preferably 1 wt.-%, further preferably 1.25 wt.-%, even further preferably 1.5 wt.-% based on the total weight of the composition.

As described above, it was surprisingly found that sertaconazol is effective already in such low amounts. These amounts are rather low, compared to common other antimycotic substances for which a sporistatic and/or sporicidal effect was already known (see Examples 1 and 2). Thus, when used in such low amounts, the risk of side effects of the use, which can also occur in non-medical areas, are particularly reduced.
Figure 1 shows the macroscopy and microscopy (HE-staining, 100-fold magnification) after infection of the full skin models with *T. rubrum, E. floccosum, S. brevicaulis,* and *C.albicans,* compared to the uninfected model and the model treated with 1 % SDS.
Figure 2 shows the influence of sertaconazol, ciclopiroolxamin and terbinafin on the viability of the full skin models.
Figure 3 shows the influence of sertaconazol, ciclopiroolxamin and terbinafin on the LDH release in the full skin models as a marker of damage in the models.
Figure 4 shows the influence of sertaconazol, ciclopiroolxamin and terbinafin on the IL-1α secretion release in the full skin models as a marker of damage in the models.
Figure 5 shows the infection of the full skin models with *T. rubrum* and the treatment with sertaconazol, ciclopiroolxamin and terbinafin over 48 h or 72 h (PAS-staining and 100-fold magnification).
Figure 6 shows the infection of the full skin models with *S*. *brevicaulis* and the treatment with sertaconazol, ciclopiroolxamin and terbinafin over 48 h or 72 h (PAS-staining and 100-fold magnification).
Figure 7 shows the infection of the full skin models with *C. albicans* and the treatment with sertaconazol, ciclopiroolxamin and terbinafin over 48 h or 72 h (PAS-staining and 100-fold magnification).

Preferred embodiments and further aspects of the present invention also emerge from the attached patent claims and the following examples, wherein the present invention is not limited to these examples.

### Examples

### Example 1: Selection of the concentration

To allow a reasonable comparison between antimycotic drugs, the concentrations to be used in the following tests was determined first. Thus, the antimycotic effect was determined by microtiter plate laser nephelometry (MLN).

MLN is a direct method for measuring light, which is scattered by particles suspended in a solution in angles of up to 90 degree. With this technique, already minor particle concentrations can be determined in a suspension. Thus, this method provides a higher sensitivity than usual transmission readers, which detect the reduction of the intensity of light passing a suspension. Further, it is a very reliable technique for application in a 96-well format. It can be used to monitor the growth of microorganisms by the turbidity of the respective medium and to examine the effect of antimicrobial substances on the growth of microorganisms. Compared to other applied methods, MLN allows high throughput screenings, incubation over a long time and *in situ* monitoring of changes in the dose-response-curves.

MLN is for example described in Wiegand C, Abel M, Ruth P, Elsner P, Hipler UC. In vitro assessment of the antimicrobial activity of wound dressings: influence of the test method selected and impact of the pH. J Mater Sci Mater Med. 2015a; 26(1):5343; Wiegand C, Abel M, Ruth P, Elsner P, Hipler UC. pH influence on antibacterial efficacy of common antiseptic substances. Skin Pharmacol Physiol. 2015b; 28(3):147-58; Wiegand C, Abel M, Ruth P, Hipler UC. Analysis of the adaptation capacity of Staphylococcus aureus to commonly used antiseptics by microplate laser nephelometry. Skin Pharmacol Physiol 2012; 25:288-297; Seyfarth F, Schliemann S, Elsner P, Hipler U.-C. Antifungal effect of high- and low-molecular-weight chitosan hydrochloride, carboxymethyl chitosan, chitosan oligosaccharide and N-acetyl-D-glucosamine against Candida albicans, Candida krusei and Candida glabrata. Int J Pharmaceut. 2008; 353:139-148; Finger S., Wiegand C., Buschmann H.-J., Hipler U.-C. Antimicrobial properties of cyclodextrin-antiseptics-complexes determined by microplate laser nephelometry and ATP bioluminescence assay. Int J Pharm 2012; 436:851-856 and Burmester A, Hipler UC, Uhrlaß S, Nenoff P, Singal A, Verma SB, Elsner P, Wiegand C. Indian Trichophyton mentagrophytes squalene epoxidase erg1 double mutants show high proportion of combined fluconazole and terbinafine resistance. Mycoses. 2020; doi: 10.1111/myc.13150.

Moreover, in most techniques, the minimum inhibitory concentration (MIC) can only be determined afterwards, whereas MLN allows the *in situ* acquisition of dose-response-curves and the simultaneous monitoring of changes in the IC₅₀.

*Candida albicans* DSM 1386, *Candida parapsilosis* DSM 5784, *Trichophyton rubrum* DSM *16111,Trichophyton interdigitale* IHEM 22939, *Trichophyton soudanense* DSM 103786, and *Trichophyton mentagrophytes* DSM 107608 were obtained from the DSMZ (German Collection of Microorganisms and Cell Cultures; Deutsche Sammlung von Mikroorganismen und Zellkulturen). *Epidermophyton floccosum* and *Scorpolariopsis brevicaulis* were obtained from a round robin test.

The yeasts were cultivated in Sabouraud-Glucose-Bouillon (SGB; Merck) for 24 h at 37°C at aerobic conditions. Dermatophytes were cultivated on Dermasel-Agarplates (Oxoid) for 3 weeks and subsequently treated with 5 mL sterile, physiological saline solution (Lot 14HD24, Fresenius Kabi Deutschland GmbH) for detaching the spores. The spore suspensions were transferred into sterile 50-ml-tubes (Greiner Bio-One), vortexed for 20 s and separated by cell sieves (40 µm, Greiner Bio-One). Subsequently, the spore number was determined via Neubauer-improved counting chambers and the spore suspension was adjusted to approximately 2 × 10³/ml in SBG (Merck) for the MLN-experiments.

Solutions of sertaconazol, ciclopiroxolamin and terbinafin were prepared as 10 µg/µL DMSO (Sigma-Aldrich). The respective test concentrations were then obtained by diluting these solutions in SGB (Merck).

The MLN-test was then performed according to NCCLS M27-A2 and DIN EN 27027 as previously described **(**Burmester A, Hipler UC, Uhrlaß S, Nenoff P, Singal A, Verma SB, Elsner P, Wiegand C. Indian Trichophyton mentagrophytes squalene epoxidase erg1 double mutants show high proportion of combined fluconazole and terbinafine resistance. Mycoses. 2020; doi: 10.111 1/myc.13150). 100 mL of the respective dilutions of sertaconazol, ciclopiroxolamin or terbinafin were added to the respective wells of a sterile, clear 96-well microplate (Greiner Bio-One) in quadruplicates together with 100 µL of the microorganism suspensions (as above). Blanks for each analysed substance concentration were added to each assay. The microtiter plates were sealed with a transparent foil (Greiner Bio-One). To allow the gas exchange, a perforation was applied to the right edge of the respective wells. The microtiter plates were then inserted in the microtiter plate laser nephelometer (NEPH-ELOstar Galaxy, BMG Labtech) and incubated at 37 °C while shaking for 48 h (yeasts) or, respectively, 120 h (mold, dermatophytes). For determining the growth of the microorganisms, the turbidity of each analysed substrate concentration was assigned to the incubation time. Subsequently, the area under curve was determined by the results and calculated as percentage of the untreated control (growth [%]). These results were used to obtain a dose-response-curve for each substance and each microorganism. The half-maximum inhibitory concentration (IC₅₀) of the substances was calculated at the applied conditions by use of a logistic adaptation function (y = A2 + (A1-A2) / (1+ (x / x0) ^ p; A1: upper limit, A2: lower limit, x0: IC50, p: gradient of the curve; OriginPro 9.1, OriginLab). Furthermore, the minimum inhibitory concentration (MIC) was determined by plating the cell suspension in the wells without visible growth in the MLN-test.

The following results were obtained:

| | **Sertaconazol** | | **Ciclopiroxolamin** | | **Terbinafin** | |
|---|---|---|---|---|---|---|
| | **IC₅₀ [µg/mL]** | **MIC [µg/mL]** | **IC₅₀ [µg/mL]** | **MIC [µg/mL]** | **IC₅₀ [µg/mL]** | **MIC [µg/mL]** |
| ***T**. **rubrum*** | 0.0028 ± 0.0003 | 1.3 | 1.02 ± 0.22 | 10.0 | 0.0015 ± 0.0002 | 0.006 |
| ***T. soudanense*** | 0.0014 ± 0.0001 | 3.1 | 1.43 ± 0.78 | 5.5 | 0.0016 ± 0.0005 | 0.015 |
| ***T. interdigitale*** | 0.39 ± 0.01 | 12.5 | 2.62 ± 0.62 | 10.0 | 0.0038 ± 0.0008 | 0.02 |
| ***T. mentagrophytes*** | 0.018 ± 0.003 | 3.8 | 2.09 ± 0.78 | 10.0 | 4.66 ± 0.04 | 20.0 |
| ***E. floccosum*** | 0.022 ± 0.003 | 1.6 | 1.46 ± 0.22 | 7.5 | 0.0021 ± 0.0001 | 0.02 |
| **S. *brevicaulis*** | 0.11 ± 0.03 | 7.5 | 4.85 ± 1.79 | 60.0 | 0.69 ± 0.03 | 40.0 |
| ***C*. *albicans*** | 0.54 ± 0.12 | 40.0 | 4.01 ± 0.71 | 40.0 | 24.17 ± 2.99 | n.d. |
| ***C*. *parapsilosis*** | 0.0025 ± 0.0001 | 20.0 | 4.73 ± 0.09 | 80.0 | 0.098 ± 0.002 | 3.8 |

### Example 2: Full skin model - infection and treatment

For an application *in vivo,* there is the additional requirement for the antimycotic substances to evoke a targeted antimycotic activity in the presence of human cells. Thus, 3D-cell-cultures such as full skin models are used to simulate the *in vivo* conditions. Such full skin models are for example described in Reddersen K, Wiegand C, Elsner P, Hipler UC. Three-dimensional human skin model infected with Staphylococcus aureus as a tool for evaluation of bioactivity and biocompatibility of antiseptics. Int J Antimicrob Agents. 2019; 54(3):283-291; Wiegand C, Fink S, Beier O, Horn K, Pfuch A, Schimanski A, Grünler B, Hipler UC, Elsner P. Dose- and Time-Dependent Cellular Effects of Cold Atmospheric Pressure Plasma Evaluated in 3D Skin Models. Skin Pharmacol Physiol. 2016;29(5):257-265. Particularly, such full skin models are made of a collagen matrix, which is colonized with primary human fibroblasts, and a fully differentiated epidermis made of primary human keratinocytes. For the experiments, the model is infected *in vitro* with spores, such that a determination of the sporistatic or sporicidal influence of the substances to be examined at *in vivo* relevant conditions is allowed. Compared to a 2D-model, the 3D full skin model has the advantage that the cells are in their natural three-dimensional surrounding and show a corresponding behaviour. Such, the antimycotic and sporistatic or sporicidal effects can be examined *in situ* and in the presence of human cells.

Additionally, the influence on the cell viability and the release of lactate dehydrogenase (LDH) was examined over the incubation time. Disruptions of the cell membranes can be measured by the release of the cytosolic enzyme LDH and be evaluated as a marker for cellular necrosis. As the biocompatibility of the material is determined by further cellular and molecular factors, the analysis of the cellular interleukin release allows the conclusion on e.g. pro-inflammatory effects of the substances, which would not be possible to be detected by sole cytotoxicity. Such interleukins are interleukins 1α, 6 or 8, which are involved in coordinating the cellular proliferation, cell migration and possible inflammatory processes. Such a method is described in, for example, Wiegand C, Hipler UC. Evaluation of biocompatibility and cytotoxicity using keratinocyte and fibroblast cultures. Skin Pharmacol Physiol 2009; 22:74-82; Wiegand C, Winter D, Hipler UC. Molecular-weight-dependent toxic effects of chitosans on the human keratinocyte cell line HaCaT. Skin Pharmacol Physiol. 2010;23(3):164-70.

The spore suspensions and the solutions of sertaconazol, ciclopiroxolamin and terbinafin were prepared as in Example 1, wherein the respective test concentrations were obtained by diluting these solutions in ultrapure water (WFI; Fresenius Kabi) instead of SGB (Merck). Full skin models were produced as previously described **[**Reddersen K, Wiegand C, Elsner P, Hipler UC. Three-dimensional human skin model infected with Staphylococcus aureus as a tool for evaluation of bioactivity and biocompatibility of antiseptics. Int J Antimicrob Agents. 2019; 54(3):283-291; Wiegand C, Fink S, Beier O, Horn K, Pfuch A, Schimanski A, Grünler B, Hipler UC, Elsner P. Dose- and Time-Dependent Cellular Effects of Cold Atmospheric Pressure Plasma Evaluated in 3D Skin Models. Skin Pharmacol Physiol. 2016;29(5):257-265].Particularly, normal human dermal fibroblasts (NHDF; Lot 1060702.1, Promocell) and normal human epidermal keratinocytes (NHEK; Lot 9100102, Promocell) were used. Fibroblasts were cultivated in Dulbecco's modified eagle medium (DMEM; Lot MC09049P, Amimed), with 2% foetal calf serum (Lot P160509; PAN), 5 ng/mL hFGF (Lot 0329586; novoprotein) and 5 µg/ml Insulin (Lot H07443P, AMIMED) at 37°C and a 5% CO₂ atmosphere. Keratinocytes were cultivated in keratinocyte medium (KBM, Lot 446M063, Promocell) at 37°C and a 5% CO₂ atmosphere. The cultivation was performed over 7 days in 75 cm² cell culture flasks (Greiner Bio-One). The cells were subsequently detached with Trypsin-EDTA (Lot 2091326; Gibco) and dissolved in the respective medium. For producing the dermis equivalent, fibroblasts were cultivated in 12-well inserts (Greiner bio-one) with DMEM (Lot MC09049P, Amimed) + 10% foetal calf serum (Lot P160509, PAN) + 1% Gentamycin (Lot 2027100, Gibco) + 150 µg/ml ascorbic acid (Lot BCBS1081V, Sigma) for 3 weeks at 3 °C and 5 % CO₂. The medium was changed all 2-3 days. Before seeding the keratinocytes for producing the dermis, the medium surrounding the insert was completely removed and the dermis upper layer was coated with fibronectin (50 µg/mL; Lot 435P078, Promocell). After 30 min of incubation of the dermis equivalent with fibronectin, the seeding of the keratinocytes was performed with 2 × 10⁵ cells / insert in KBM (Lot 446M063, Promocell) + 5% FKS (Lot P160509, PAN) + 1% Gentamycin (Lot 2027100, Gibco). Subsequently, the skin models were incubated for 45 min in the incubator before the models were flooded with KBM + 5% FKS + 1% Gentamycin. The skin models were cultivated for 7 days by submersion in the medium with decreasing foetal calf serum concentration (5 %, 2 %, 0 %). The medium was changed all 2-3 days. After the cultivation by submersion, the skin models were further cultivated for 12 days at the air-medium interface (airlift cultivation). For this purpose, the skin models were transferred into ThinCert 12-well plates (Greiner Bio-One), including Greens-medium (1:1 DMEM + DMEM HAMS-F12 (Lot 2063525; life technologies) + 5% foetal calf serum + 1% Gentamycin + 10 ng/mL hEGF (Lot 4020302; Promocell) + 0.33 µg/mL Hydrokortison (Lot SLBL1760V; Sigma) + 10-4 M adenine (Lot SLBF5842V, Sigma) + 5 µg/mL Insulin (Lot QC-31B19F05; PeloBiotech) + 5 µg/mL Transferrin (Lot 2621-20; BBI-Solution) + 2 × 10⁻⁷ M Triiodothyronin (Lot SLFB1732V, Sigma) + 1,88 mM CaCl2 (Lot 00416, Serumwerk Bernburg). The medium was changed all 2-3 days.

For the infection, collagen pellicles were produced from 6 mg/ml collagen suspension (Fraunhofer Institut) and were loaded with 25 µl spore suspension of the fungi (10⁴/mL, *C*. *albicans:* 2 × 10²/mL). Subsequently, the skin models were placed onto the spore-loaded collagen pellicles and cultivated for further 72 h.

For treating the skin models with the antimycotic substances to be tested (sertaconazol, ciclopiroxolamin und terbinafin), the Greens-medium was replaced with fresh medium and 25 µl of the substance solution (for concentrations see the table below) were directly added to the infected full skin models. The negative control was treated with 25 µl ultrapure water (WFI; Fresenius Kabi). The positive control was treated with 25 µl 1 % SDS (Roth). The samples were incubated for 48 h and 72 h at 37 °C and 5 % CO₂. The following concentrations of sertaconazole, ciclopiroxolamine and terbinafine were used for the treatment of infected full skin models:

| | Sertaconazol [µg/mL] | Ciclopiroxolamin [µg/mL] | Terbinafin [µg/mL] |
|---|---|---|---|
| *T. rubrum* | 0.025 | 400 | 0.050 |
| *E. floccosum* | 0.010 | 400 | 0.200 |
| *S*. *brevicaulis* | 100 | 400 | 100 |
| *C. albicans* | 800 | 400 | 1600 |

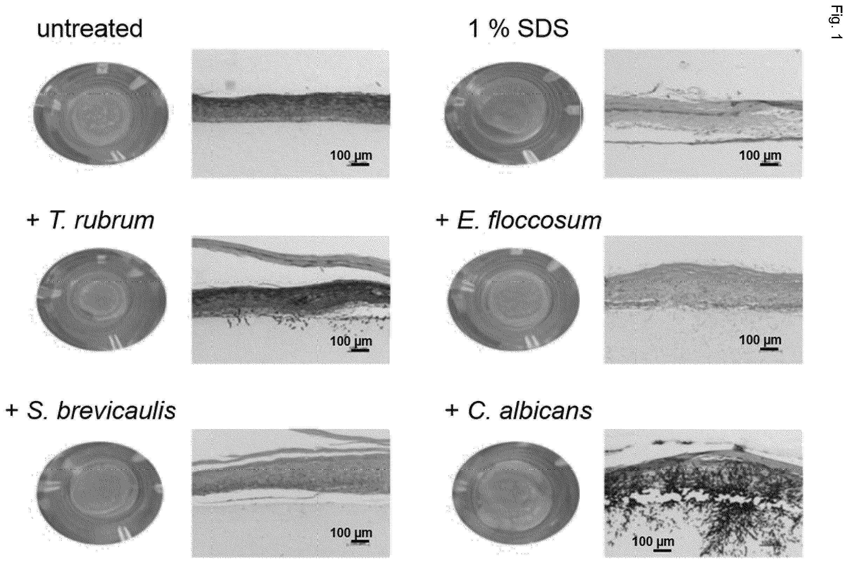

An infection of the full skin models with *T. rubrum, E. floccosum, S. brevicaulis* and *C*. *albicans* could be clearly confirmed with HE- and PAS-staining (Fig. 1). While the two dermatophytes and the mold only led to minor changes in the skin layers after 48 hours, the yeast was widespread and could already be detected macroscopically on the skin model as white floccus (Fig. 1).

The cell viability was determined based on the luminometric ATP measurement with the ATPLite^{™}-M Assay (PerkinElmer) as previously reported **[**Reddersen K, Wiegand C, Elsner P, Hipler UC. Three-dimensional human skin model infected with Staphylococcus aureus as a tool for evaluation of bioactivity and biocompatibility of antiseptics. Int J Antimicrob Agents. 2019; 54(3):283-291; Wiegand C, Fink S, Beier O, Horn K, Pfuch A, Schimanski A, Grünler B, Hipler UC, Elsner P. Dose- and Time-Dependent Cellular Effects of Cold Atmospheric Pressure Plasma Evaluated in 3D Skin Models. Skin Pharmacol Physiol. 2016;29(5):257-265]. The assay is based on the production of light, originating from the reaction of ATP with luciferase and D-luciferin. The emitted light is directly proportional to the ATP-concentration. Particularly, for each full skin model, 500 µl of the cell lysate solution were added to each well and it was stirred in an orbital shaker for 15 min at 700 rpm. Subsequently, 100 µl were transferred into a white 96-well microtiter plate and 100 µl substrate solution (luciferin/luciferase) were added to each well. The samples were again shaken for 5 min, and then incubated for 10 min in the dark and the luminescence was measured in the microplate luminometer LUMlstar Galaxy (BMG LabTechnologies GmbH). The ATP concentrations were determined based on an ATP standard curve.

Measuring the LDH release was accomplished with the Cytotoxicity Detection Kit (Roche Diagnostics) as previously described **[**Reddersen K, Wiegand C, Elsner P, Hipler UC. Three-dimensional human skin model infected with Staphylococcus aureus as a tool for evaluation of bioactivity and biocompatibility of antiseptics. Int J Antimicrob Agents. 2019; 54(3):283-291; Wiegand C, Fink S, Beier O, Horn K, Pfuch A, Schimanski A, Grünler B, Hipler UC, Elsner P. Dose- and Time-Dependent Cellular Effects of Cold Atmospheric Pressure Plasma Evaluated in 3D Skin Models. Skin Pharmacol Physiol. 2016;29(5):257-265]. Thereby, a colorimetric assay is performed for quantifying the cell lysis, based on the measurement of activity of LDH, which is released from the cytosol to the medium in case the cell membrane is damaged. Particularly, 100 µl of culture supernatants of the full skin models were in duplicates transferred to a transparent 96-well microtiter plate (Greiner Bio-One) and 100 µl tetrazoline dye were added per well. Subsequently, the samples were incubated for 30 at room temperature in the dark. The absorption at 490 nm was measured in the photometer POLARstar Galaxy (BMG Labtech).

For measuring the interleukin secretion of the full skin models during the treatment with the samples as described above, was performed using the respective ELISAs for IL-1α (R&D Systems), IL-6 (Mabtech) and IL-8 (R&D Systems). The ELISAs were performed according to the manufacturer's instructions. The culture supernatants of the full skin models were used without dilution.

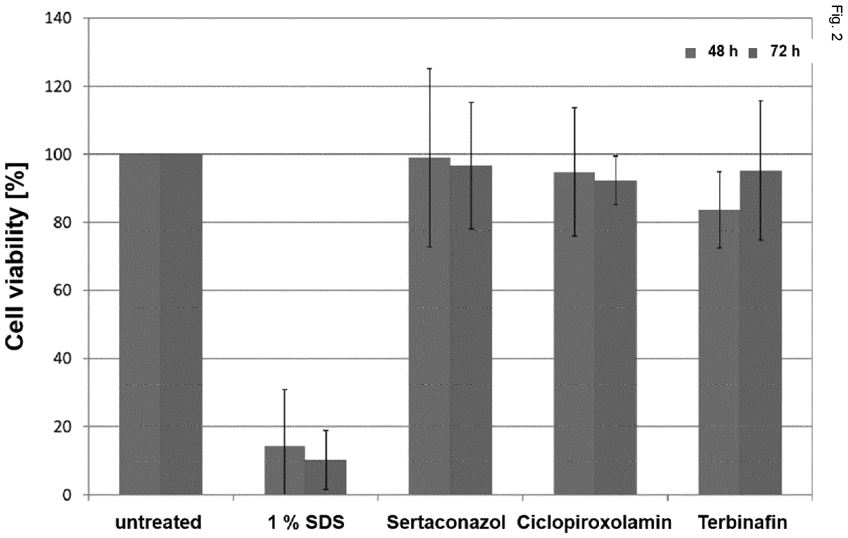

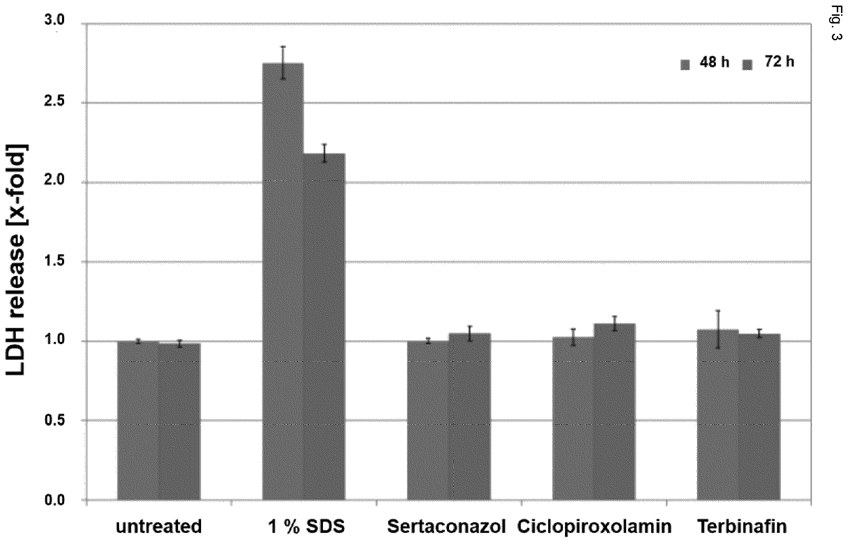

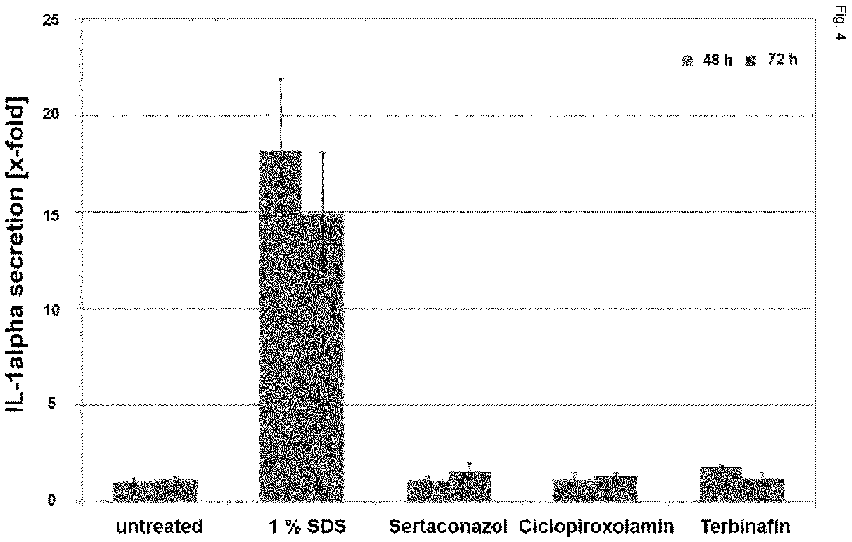

The control with 1 % SDS resulted in a clear disruption of the full skin models. The applied concentrations of sertaconazol, ciclopiroxolamin und terbinafin (determined as described above) did not have a negative effect on the cell viability of the full skin models (Fig. 2). The measured values for the cell viability did not drop below the minimum value of 70 % (set according to DIN EN ISO 10993-5). The sample with 1 % SDS resulted in a significant reduction of cell viability of the full skin models below the minimum value and to approximately 20%. Furthermore and in contrast to the 1 % SDS sample, no significant secretion of LDH of IL-1α was observed (Figs. 3, 4).

Infection of the skin models only resulted in a reduction of the cell viability and a slight increase of LDH release and secretion of IL-1α for *S. brevicaulis* and *C. albicans.* The dermatophytes *T. rubrum* and *E. floccosum* did not have an effect on these markers in the skin models. Thus, the secretion of IL-6 and IL-8 was also examined in the full skin models after infection. However, these pro-inflammatory markers were only induced by *C*. *albicans.* Consequently, the evaluation of a disruption of the full skin models by an infection and the effectiveness of a treatment was mainly based on the histological results.

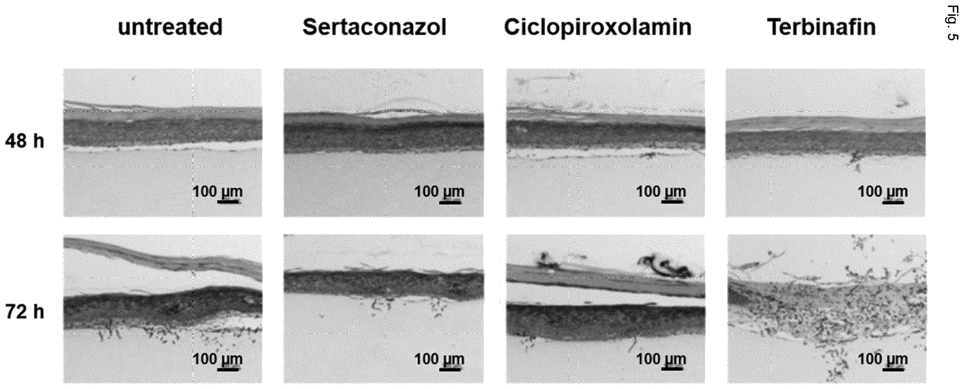

It was surprisingly found that sertaconazol could inhibit the outgrowth of the spores of *T. rubrum* for 48 h (Fig. 5) and reduce the outgrowth at 72 h. Ciclopiroxolamin could also reduce the spread of *T. rubrum* but could not inhibit it. In contrast, terbinafin did not have any effect up to 72 h after application and the histological results were comparable to the infected and untreated control.

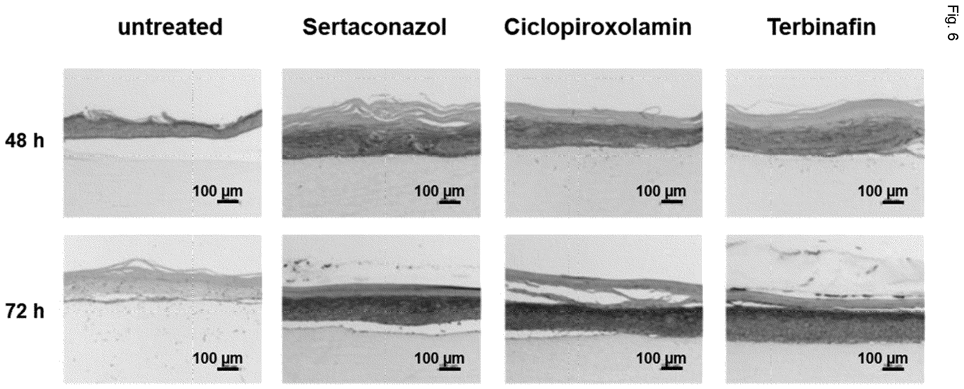

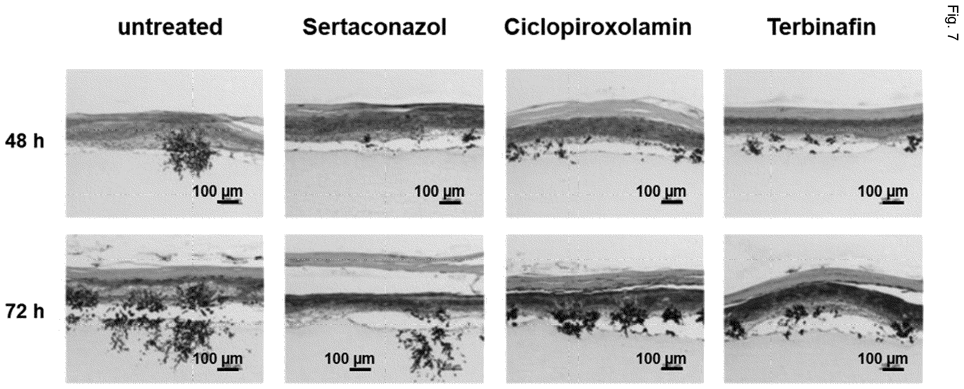

The spread of *E*. *floccosum* was rather low in the full skin model and was mainly observed in the deeper layers. The outgrowth of *E*. *floccosum* could be inhibited by sertaconazol and could be only reduced by ciclopiroxolamin and terbinafin. The same applies for *S*. *brevicaulis* (Fig. 6). The outgrowth of *C*. *albicans* could be clearly reduced by sertaconazol over 72 h, whereas the treatment with ciclopiroxolamin or terbinafin had a much lower effect (Fig. 7).

## Claims

1. Sertaconazol, or a salt thereof, preferably sertaconazol nitrate, for use in the prevention and/or treatment of a fungal infection, wherein the prevention and/or treatment comprises or is providing a sporistatic and/or sporicidal effect against the spores of the fungus causing the fungal infection.

2. Sertaconazol, or a salt thereof, preferably sertaconazol nitrate, for use according to claim 1, wherein the fungus causing the fungal infection is selected from the group consisting of the genera *Trichophyton, Epidermophyton, Microascus* or *Candida.*

3. Sertaconazol, or a salt thereof, preferably sertaconazol nitrate, for use according to any of claim 1 or 2, wherein the fungus causing the fungal infection is selected from the group consisting of *Trichophyton rubrum, Trichophyton soudanense, Trichophyton interdigitale, Trichophyton mentagrophytes, Epidermophyton floccosum, Scopulariopsis brevicaulis, Candida albicans* and *Candida parapsilosis.*

4. Sertaconazol, or a salt thereof, preferably sertaconazol nitrate, for use according to any of the preceding claims, wherein the fungus causing the fungal infection is selected from the group consisting of *Trichophyton mentagrophytes, Scopulariopsis brevicaulis, Candida albicans* and *Candida parapsilosis.*

5. Sertaconazol, or a salt thereof, preferably sertaconazol nitrate, for use according to any of the preceding claims, wherein the prevention and/or treatment comprises applying a composition comprising sertaconazol, or a salt thereof, preferably sertaconazol nitrate, in a concentration of at least 0.0001 wt.-%, preferably at least 0.001 wt.-%, particularly preferably at least 0.01 wt.-%, based on the total weight of the composition.

6. Sertaconazol, or a salt thereof, preferably sertaconazol nitrate, for use according to any of the preceding claims, wherein the fungal infection is an infection of the skin and/or a nail of a subject.

7. Sertaconazol, or a salt thereof, preferably sertaconazol nitrate, for use according to any of the preceding claims, wherein the prevention and/or treatment comprises a local application, preferably a dermal application or an application on the nail of a subject, of sertaconazol, or a salt thereof, preferably sertaconazol nitrate.

8. Non-therapeutic use of sertaconazol, or a salt thereof, preferably sertaconazol nitrate, for providing a sporistatic and/or sporicidal effect against the spores of a fungus.

9. Non-therapeutic use according to claim 8, wherein the fungus is selected from the group consisting of fungi.

10. Non-therapeutic use according to claim 8 or 9, wherein the fungus is selected from the group consisting of the genera *Trichophyton, Epidermophyton, Microascus* or *Candida.*

11. Non-therapeutic use according to any of claims 8 to 10, wherein the fungus is selected from the group consisting of *Trichophyton rubrum, Trichophyton soudanense, Trichophyton interdigitale, Trichophyton mentagrophytes, Epidermophyton floccosum, Scopulariopsis brevicaulis, Candida albicans* and *Candida parapsilosis.*

12. Non-therapeutic use according to any of claims 8 to 11, wherein the fungus is selected from the group consisting of *Trichophyton mentagrophytes, Scopulariopsis brevicaulis, Candida albicans* and *Candida parapsilosis.*

13. Non-therapeutic use according to any of claims 8 to 12, wherein sertaconazol or a salt thereof, preferably sertaconazol nitrate, is present in a composition in a concentration of at least 0.0001 wt.-%, preferably at least 0.001 wt.-%, particularly preferably at least 0.01 wt.-%, based on the total weight of the composition.

## Patentansprüche

1. Sertaconazol oder Salz davon, vorzugsweise Sertaconazolnitrat, zur Anwendung in der Vorbeugung und/oder Behandlung einer Pilzinfektion, wobei die Vorbeugung und/oder Behandlung das Vermitteln einer sporistatischen und/oder sporiziden Wirkung gegen die Sporen des Pilzes, der die Pilzinfektion verursacht, enthält oder ist.

2. Sertaconazol oder Salz davon, vorzugsweise Sertaconazolnitrat, zur Anwendung nach Anspruch 1, wobei der Pilz, der die Pilzinfektion verursacht, ausgewählt ist aus der Gruppe bestehend aus den Genera *Trichophyton, Epidermophyton, Microascus* oder *Candida.*

3. Sertaconazol oder Salz davon, vorzugsweise Sertaconazolnitrat, zur Anwendung nach Anspruch 1 oder 2, wobei der Pilz, der die Pilzinfektion verursacht, ausgewählt ist aus der Gruppe bestehend aus *Trichophyton rubrum, Trichophyton soudanense, Trichophyton interdigitale, Trichophyton mentagrophytes, Epidermophyton floccosum, Scopulariopsis brevicaulis, Candida albicans* und *Candida parapsilosis.*

4. Sertaconazol oder Salz davon, vorzugsweise Sertaconazolnitrat, zur Anwendung nach einem der vorangehenden Ansprüche, wobei der Pilz, der die Pilzinfektion verursacht, ausgewählt ist aus der Gruppe bestehend aus *Trichophyton mentagrophytes, Scopulariopsis brevicaulis, Candida albicans* und *Candida parapsilosis.*

5. Sertaconazol oder Salz davon, vorzugsweise Sertaconazolnitrat, zur Anwendung nach einem der vorangehenden Ansprüche, wobei die Vorbeugung und/oder Behandlung das Auftragen einer Zusammensetzung enthält, die Sertaconazol oder ein Salz davon, vorzugsweise Sertaconazolnitrat, in einer Konzentration von mindestens 0,0001 Gew.-%, vorzugsweise mindestens 0,001 Gew.-%, besonders bevorzugt mindestens 0,01 Gew.-%, bezogen auf das Gesamtgewicht der Zusammensetzung, enthält.

6. Sertaconazol oder Salz davon, vorzugsweise Sertaconazolnitrat, zur Anwendung nach einem der vorangehenden Ansprüche, wobei die Pilzinfektion eine Infektion der Haut und/oder eines Nagels eines Subjekts ist.

7. Sertaconazol oder Salz davon, vorzugsweise Sertaconazolnitrat, zur Anwendung nach einem der vorangehenden Ansprüche, wobei die Vorbeugung und/oder Behandlung eine lokale Applikation von Sertaconazol oder eines Salzes davon, vorzugsweise Sertaconazolnitrat, vorzugsweise eine dermale Applikation oder eine Applikation auf den Nagel eines Subjekts, enthält.

8. Nicht-therapeutische Verwendung von Sertaconazol oder einem Salz davon, vorzugsweise Sertaconazolnitrat, zum Vermitteln einer sporistatischen und/oder sporiziden Wirkung gegen die Sporen eines Pilzes.

9. Nicht-therapeutische Verwendung nach Anspruch 8, wobei der Pilz ausgewählt ist aus der Gruppe bestehend aus Pilzen.

10. Nicht-therapeutische Verwendung nach Anspruch 8 oder 9, wobei der Pilz ausgewählt ist aus der Gruppe bestehend aus den Genera *Trichophyton, Epidermophyton, Microascus* oder *Candida.*

11. Nicht-therapeutische Verwendung nach einem der Ansprüche 8 bis 10, wobei der Pilz ausgewählt ist aus der Gruppe bestehend aus *Trichophyton rubrum, Trichophyton soudanense, Trichophyton interdigitale, Trichophyton mentagrophytes, Epidermophyton floccosum, Scopulariopsis brevicaulis, Candida albicans* und *Candida parapsilosis.*

12. Nicht-therapeutische Verwendung nach einem der Ansprüche 8 bis 11, wobei der Pilz ausgewählt ist aus der Gruppe bestehend aus *Trichophyton mentagrophytes, Scopulariopsis brevicaulis, Candida albicans* und *Candida parapsilosis.*

13. Nicht-therapeutische Verwendung nach einem der Ansprüche 8 bis 12, wobei Sertaconazol oder ein Salz davon, vorzugsweise Sertaconazolnitrat, in einer Zusammensetzung in einer Konzentration von mindestens 0,0001 Gew.-%, vorzugsweise mindestens 0,001 Gew.-%, besonders bevorzugt mindestens 0,01 Gew.-%, bezogen auf das Gesamtgewicht der Zusammensetzung, vorhanden ist.

## Revendications

1. Sertaconazole ou sel de celui-ci, de préférence nitrate de sertaconazole, pour une utilisation dans la prévention et/ou le traitement d'une infection fongique, la prévention et/ou le traitement comprenant ou consistant à conférer un effet sporistatique et/ou sporicide contre les spores du champignon qui provoque l'infection fongique.

2. Sertaconazole ou sel de celui-ci, de préférence nitrate de sertaconazole, pour une utilisation selon la revendication 1, le champignon qui provoque l'infection fongique étant choisi dans le groupe constitué par les genres *Trichophyton, Epidermophyton, Microascus* ou *Candida.*

3. Sertaconazole ou sel de celui-ci, de préférence nitrate de sertaconazole, pour une utilisation selon la revendication 1 ou 2, le champignon qui provoque l'infection fongique étant choisi dans le groupe constitué par *Trichophyton rubrum, Trichophyton soudanense, Trichophyton interdigitale, Trichophyton mentagrophytes, Epidermophyton floccosum, Scopulariopsis brevicaulis, Candida albicans* et *Candida parapsilosis.*

4. Sertaconazole ou sel de celui-ci, de préférence nitrate de sertaconazole, pour une utilisation selon l'une des revendications précédentes, le champignon qui provoque l'infection fongique étant choisi dans le groupe constitué par *Trichophyton mentagrophytes, Scopulariopsis brevicaulis, Candida albicans* et *Candida parapsilosis.*

5. Sertaconazole ou sel de celui-ci, de préférence nitrate de sertaconazole, pour une utilisation selon l'une des revendications précédentes, la prévention et/ou le traitement comprenant l'application d'une composition qui contient du sertaconazole ou un sel de celui-ci, de préférence du nitrate de sertaconazole, en une concentration d'au moins 0,0001 % en poids, de préférence d'au moins 0,001 % en poids, de manière particulièrement préférée d'au moins 0,01 % en poids, par rapport au poids total de la composition.

6. Sertaconazole ou sel de celui-ci, de préférence nitrate de sertaconazole, pour une utilisation selon l'une des revendications précédentes, l'infection fongique étant une infection de la peau et/ou d'un ongle d'un sujet.

7. Sertaconazole ou sel de celui-ci, de préférence nitrate de sertaconazole, pour une utilisation selon l'une des revendications précédentes, la prévention et/ou le traitement comprenant une application locale de sertaconazole ou d'un sel de celui-ci, de préférence de nitrate de sertaconazole, de préférence une application dermique ou une application sur l'ongle d'un sujet.

8. Utilisation non thérapeutique de sertaconazole ou d'un sel de celui-ci, de préférence de nitrate de sertaconazole, pour conférer un effet sporistatique et/ou sporicide contre les spores d'un champignon.

9. Utilisation non thérapeutique selon la revendication 8, le champignon étant choisi dans le groupe constitué par les champignons.

10. Utilisation non thérapeutique selon la revendication 8 ou 9, le champignon étant choisi dans le groupe constitué par les genres *Trichophyton, Epidermophyton, Microascus* ou *Candida.*

11. Utilisation non thérapeutique selon l'une des revendications 8 à 10, le champignon étant choisi dans le groupe constitué par *Trichophyton rubrum, Trichophyton soudanense, Trichophyton interdigitale, Trichophyton mentagrophytes, Epidermophyton floccosum, Scopulariopsis brevicaulis, Candida albicans* et *Candida parapsilosis.*

12. Utilisation non thérapeutique selon l'une des revendications 8 à 11, le champignon étant choisi dans le groupe constitué par *Trichophyton mentagrophytes, Scopulariopsis brevicaulis, Candida albicans* et *Candida parapsilosis.*

13. Utilisation non thérapeutique selon l'une des revendications 8 à 12, du sertaconazole ou un sel de celui-ci, de préférence du nitrate de sertaconazole, étant présent dans une composition en une concentration d'au moins 0,0001 % en poids, de préférence d'au moins 0,001 % en poids, de manière particulièrement préférée d'au moins 0,01 % en poids, par rapport au poids total de la composition.
